# EUROPEAN PATENT SPECIFICATION

(11) **EP 3 369 901 B1**
(45) Date of publication and mention of the grant of the patent: **16.10.2019**
(21) Application number: 17305230.9
(22) Date of filing: 03.03.2017
(51) Int. Cl.: F01N 3/20, B01D 35/027, B01D 35/30, B01D 29/07, F01N 11/00

(54) **UREA SENSOR PROTECTION ASSEMBLY AND UREA SENSOR SYSTEM**
HARNSTOFFSENSORSCHUTZANORDNUNG UND HARNSTOFFSENSORSYSTEM
ENSEMBLE DE PROTECTION DE CAPTEUR D'URÉE ET SYSTÈME DE CAPTEUR D'URÉE

(43) Date of publication of application: 05.09.2018
(73) Proprietor: MEAS France, 31027 Toulouse Cedex 3 (FR)
(72) Inventor: CASTANDET, Armand, 31500 Toulouse (FR); LEGER, Vincent, 31200 Toulouse (FR); TOULON-MEEKHUN, Dariga, 31520 Ramonville (FR)
(74) Representative: Grünecker Patent- und Rechtsanwälte PartG mbB

(56) References cited:
- EP-A1- 2 913 494
- WO-A1-2011/078692
- DE-A1-102015 224 093
- US-A1- 2015 033 700

## Description

The invention is related to a urea sensor protection assembly for protecting at least one fluid sensor of a urea sensor system against bubbles and/or particles.

Urea sensor systems and the corresponding sensor protection assemblies are known in the prior art and are generally used in tanks for urea solutions such as diesel exhaust fluid (DEF) for diesel engines. The fluid sensors are typically used for monitoring certain properties of the urea solution in order to analyze the quality of the solution. However, certain circumstances may negatively influence the measurements. A first problem may be that urea solution tends to create bubbles during movement of the solution. For example, during filling of the urea tank, a large amount of gas bubbles may be created. These bubbles may influence measurements of the fluid sensor. Other known problems which may disturb measurements of the fluid sensor are particles, for example, particles formed by debris or frozen urea solution.

Exemplary examples of urea sensor systems are known from documents US 2015/033700 A1, DE 10 2015 224093 A1, EP 2 913 494 A1 and WO 2011/078692 A1.

It is therefore an object of the invention to provide a urea sensor protection assembly and a urea sensor system which allow to effectively protect a fluid sensor of a sensor system.

For the urea sensor protection assembly according to the invention, this object is reached in that the assembly comprises an inner cage for supporting the at least one fluid sensor and an outer cage comprising at least one filter member for blocking air bubbles and/or allowing fluid to pass, wherein the inner cage is, in an assembled state, at least partially arranged in an inner volume of an outer cage, and wherein the inner cage and the outer cage each comprise at least one separate fixation means for separately fixating the corresponding cage to a common holder. The solution according to the invention allows to effectively protect a fluid sensor due to the filter members which allow only fluids to reach the sensor but which prevent air bubbles and/or debris to reach the sensor. Further, since the inner cage and the outer cage are fixated separately to the common holder of the sensor system, the weight of each cage is carried by its own fixation means. Consequently, the weight of the outer cage does not have to be carried by the inner cage and/or the fluid sensor itself. Thereby, stress on the sensor and/or the inner cage due to vibrations of the urea sensor system is effectively reduced.

The invention further relates to a urea sensor arrangement which comprises, additionally to the urea sensor protection assembly according to the invention, at least one fluid sensor which is arranged inside the inner cage. Such a urea sensor arrangement will benefit from the advantages of the urea sensor protection assembly as described above.

The invention finally relates to a urea sensor system which comprises at least one tubular member, in particular a heating tube for being arranged in a fluid tank, in particular a urea solution tank. The urea sensor system according to the invention further comprises at least one urea sensor protection assembly as described above, wherein the inner cage and the outer cage of the urea sensor protection assembly are, in particular separately, fixated to the at least one tubular member. The urea sensor system according to the invention also benefits from the advantages of the urea sensor protection assembly as described above.

In the following, further improvements of the invention are described. The additional improvements may be combined independently of each other, depending on whether a particular advantage of a particular improvement is needed in a specific application.

According to a first advantageous improvement of the urea sensor protection assembly according to the invention, the outer cage may be composed of at least one upper cover and at least one lower cover, the at least one upper cover and the at least one lower cover jointly forming a receptacle for the inner cage. Providing an outer cage which consists of at least two parts may facilitate the assembling of the urea sensor protection assembly. Further, manufacturing the parts of the outer cage may also be facilitated.

In order to provide sufficient flow of liquid into and out of the outer cage, the at least one upper cover and the at least one lower cover may each comprise at least one filter member. In particular, the at least one upper cover may comprise at least one filter member in an upper wall and the at least one lower cover preferably comprises at least one filter member in a bottom or lower wall. The directions up and down are thereby related with respect to a bubble ascension direction which refers to the upward direction and the opposite direction which is identical to a gravitational direction and which defines the downward direction.

If a fluid sensor is arranged in the urea sensor protection assembly, then it is preferably basically arranged between the at least one filter member in the upper cover and the at least one filter member in the lower cover.

For forming a stable outer cage, the at least one upper cover and the at least one lower cover are preferably fixated to each other by integrally formed fixation means in the assembled state. For example, both covers may be provided with integrally formed fixation means which are formed complementary to each other. Alternatively, or additionally, the covers may be fixated to each other by additional fixation means such as screws.

According to another advantageous improvement of the urea sensor protection assembly according to the invention, the inner cage may be composed of at least one upper part and at least one lower part which are preferably formed complementary to each other. Forming the inner cage of at least two parts may also here facilitate the production of the inner cage itself and also the assembly of the parts to form the inner cage. Further, inserting a fluid sensor into the inner cage may be facilitated. The at least one upper part and the at least one lower part are preferably provided with through holes for letting fluid pass from the outer cage through the inner cage towards a fluid sensor.

For effectively supporting a fluid sensor, the at least one upper part preferably comprises at least one sensor retainer for fixating the fluid sensor in the assembled state, in particular between itself and the at least one lower part. The at least one sensor retainer is preferably provided with through holes for letting fluid pass to and from the fluid sensor. The at least one sensor retainer is preferably arranged between at least one filter member of the upper cover of the outer cage and the fluid sensor in the assembled state.

At least one fixation means from the inner cage and/or the outer cage is preferably formed clamp like for being fixated to a tubular part of the sensor system, in particular to a heating tube. Consequently, the at least one fixation means is preferably formed complementary to the tubular part. For example, if the tubular part has a circular cross-section, the at least one fixation means may have a shape that at least partly resembles the inner side of a cylindrical shell.

In order to allow bubbles which may be formed inside the urea sensor protection assembly to leave the assembly, the urea protection assembly is preferably provided with at least bubble outlet opening.

In order to allow bubbles to easily leave the urea sensor protection assembly, the at least one bubble outlet opening is preferably arranged above the inner cage, in particular above the fluid sensor, seen in a bubble ascension direction. More preferably, the at least one bubble outlet opening is arranged in the upper most part of the outer cage, seen in the bubble ascension direction.

For guiding bubbles towards the at least one bubble opening, the upper cover may have an inclined upper wall which rises in the bubble ascension direction towards the at least one bubble outlet opening. Alternatively, the upper cover may be oriented horizontally, or perpendicular to the bubble ascension direction.

The at least one bubble outlet opening may be formed by a gap between the inner cage and the outer cage. Thereby, at least one bubble outlet opening may easily be formed.

In order to easily define the size of the opening and to keep the at least one bubble outlet opening open, the inner cage and the outer cage may be spaced apart by at least one spacer. By this at least one spacer, the at least one bubble outlet opening may be formed. The at least one spacer is preferably formed integrally with the inner and/or the outer cage. The at least one spacer may also keep two bubble outlet openings open when it is arranged between said two openings.

A gap between the inner and outer cage may easily be formed between the upper cover of the outer cage and the upper part of the inner cage. These parts may be separated by the above mentioned spacers.

The properties of the filter members and the size of the bubble outlet opening are preferably chosen such that fluid may pass through the outer and inner cage, but at a moderate flow rate. This helps in preventing too strong thermal gradients inside the assembly. If, for example, the temperature of a fluid in the fluid tank changes rapidly, the fluid inside the assembly will follow the temperature changes slowly in order to even out peaks in the temperature profile.

The outer cage is preferably closed from at least three sides, wherein the inner cage may be inserted in another side. In particular, two sides of the outer cage may comprise a filter member, in particular the upper and the lower side. From the remaining four sides, three sides are preferably closed. The fourth side may be opened in order to receive the inner cage. This allows a stable and compact structure.

Said at least three sides which are preferably closed are more preferably formed by solid side walls. This is particularly advantageous if an optical and/or light-sensitive sensor is used. Solid side walls may prevent light from distorting the measurements made by a fluid sensor inside the inner cage and thus may improve the measurements of the fluid sensor. The arrangement may further be formed such that no reflective, such as metal and or/metalized parts in the outer cage face the fluid sensor, in particular, a light-sensitive and/or optical sensor such that the optical sensor of the fluid sensor is not disturbed by light reflections on said metal parts, for example by external light sources or by a light source of the fluid sensor itself.

The at least one fluid sensor of the urea sensor arrangement is preferably an optical and/or light-sensitive sensor, in particular working in the near infrared region. Additionally or alternatively, the fluid sensor may be an ultrasonic sensor. The at least one fluid sensor may additionally or alternatively be formed by any appropriate sensor depending on the quantities to be measured.

The urea sensor system according to the invention may further be improved in that the at least one fixation means of the inner cage abuts the at least one fixation means of the outer cage on the tubular member. Thereby, the inner cage and the outer cage may be aligned with respect to each other during assembly.

In order to prevent distortions of the measurements of the at least one fluid sensor by thermal gradients inside the fluid, the inner cage may be arranged between two parallel sections of the at least one tubular member. In particular if the tubular member is a heating tube, this may have an influence on the inner cage. Further, the urea sensor system assembly is preferably spaced apart from a loop section of the heating tube in order to prevent too strong heating of the sensor by the tube.

In the following, the invention and its improvements are described in greater detail using an exemplary embodiment and with reference to the Figures. As described above, the various features shown in the embodiment may be used independently of each other in specific applications.

In the following Figures, elements having the same function and/or the same structure will be referenced by the same reference signs.

In the drawings:
- Fig. 1: shows a sensor system according to the invention in a pre-assembled state in an exploded view;
- Fig. 2: shows the system of Fig. 1 in a state in which the outer cage is lifted away from the remaining system for better visibility;
- Fig. 3: shows the system in an assembled state;
- Fig. 4: shows the system as shown in Fig. 3 from a different point of view; and
- Fig. 5: shows a part of Fig. 4 in an enlarged view.

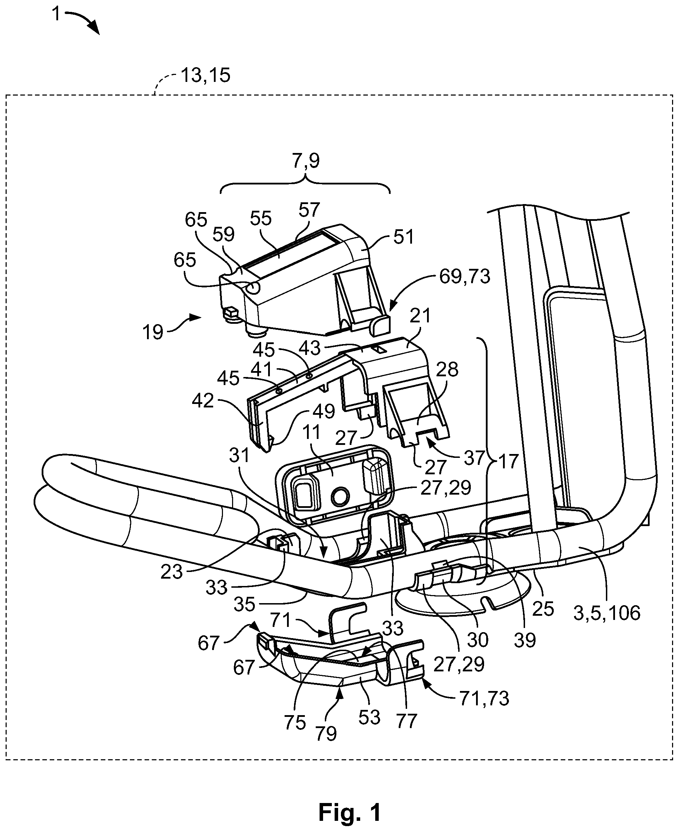

A urea sensor system 1 according to the invention is shown in Fig. 1 in an exploded view. The urea sensor system 1 comprises a tubular member 3 which is, just by way of example, a heating tube 5. Alternatively, the tubular member 3 may also be a suction tube or any other tubular member.

The urea sensor system 1 further comprises a urea sensor arrangement 7 which is connectable to the tubular member 3.

The urea sensor arrangement 7 is composed of a urea sensor protection assembly 9 according to the invention and a fluid sensor 11. By way of example, the fluid sensor 11 is an optical sensor, in particular a near infrared (NIR) sensor. Alternatively, the fluid sensor 11 may also be a sensor working with visible light or a sensor working with other techniques such as ultrasonic sound or other means.

The urea sensor system 1 may be used in a fluid tank 13 which is indicated by the dashed line. The fluid tank 13 may in particular be a urea tank 15 which can be used for industrial and commercial transportation and for automotive applications, including commercial vehicles such as trucks, tractors and any other vehicles with combustion engines, for example, to provide diesel exhaust fluid (DEF) for diesel engines. Applications include any kind of mobile and/or stationary internal combustion engine.

In the following, the urea sensor protection assembly 9 and its components are described in greater detail. The urea sensor protection assembly 9 comprises an inner cage 17 for receiving the fluid sensor 11 and an outer cage 19.

Both the inner cage 17 and the outer cage 19 are each composed of two parts.

The inner cage 17 is composed of an upper part 21 and a lower part 23, which are each formed complementary to each other. The lower part 23 may be formed integrally with a bottom plate 25 of the urea sensor system 1. The lower part 23 comprises lower fixation parts 30. The lower fixation parts 30 of the lower part 23 are formed complementary to the tubular member 3. Consequently, the lower fixation parts 30 form receptacles 29 which, on their inner sides, have an overall cylindrical shape.

The lower part 23 is further provided with a fluid sensor receptacle 31 for receiving the fluid sensor 11. The fluid sensor 11 may be inserted into the fluid sensor receptacle 31 until it is seated in the receptacle 31. Thereby, it may be supported by inner walls 33 of the receptacle 31.

A bottom wall 35 of the lower part 23 which forms a lower end of the fluid sensor receptacle 31, may be provided with through holes (not shown) for letting fluid pass into the fluid sensor receptacle 31 in order to be analyzed by the fluid sensor 11.

The upper part 21 comprises upper fixation parts 28 which are formed complementary to the lower fixation parts 30 of the lower part 23. The upper fixation parts 28 are shaped complementary to the tubular member 3. Consequently, the upper fixation parts 28 of the upper part 21 resemble the shape of a cylindrical shell. Each of the fixation means 27 of the upper part 21 is provided with a recess 37 into which a protrusion 39 which is arranged on each of the lower fixation parts 30 of the lower part 23, can protrude in an assembled state.

In an assembled state A (not shown yet), the upper fixation parts 28 and the lower fixation parts 30 together, form fixation means 27 of the inner cage 17. It should be mentioned that this arrangement may also be the other way around, e.g. the recess 37 may be arranged in the lower fixation parts 30 of the lower part 23 and the protrusion 39 may be arranged in the upper fixation parts 28 of the upper part 21.

The upper part 21 is further provided with a sensor retainer 41. The sensor retainer 41 is adapted for fixating the fluid sensor 11 in the assembled state. The sensor retainer 41 is formed by an upper wall 43 of the upper part 21. The sensor retainer 41, more preferably the whole upper wall 43 may comprise through holes 45 for letting fluid pass to the fluid sensor 11. The through holes 45 are indicated by circles. However, the through holes 45 may have any other appropriate shape and may be arranged in any appropriate number.

The sensor retainer 41 extends away from the side of the upper part 21 which carries the fixation means 27 in a bar-like shape. On its distal end, the sensor retainer 41 comprises an arm 47 with a latching hook 49, which is adapted for being latched to the lower part 23. The lower part 23 is preferably provided with complementary means for receiving the latching hook 49.

The outer cage 19 is composed of an upper cover 51 and a lower cover 53 which are formed complementary to each other. The upper cover 51 comprises a filter member 55 which is preferably semi-permeable and formed by a mesh material. The filter member 55 is preferably adapted for letting fluid, in particular a urea solution pass, whereas air bubbles and/or particles such as debris or ice are blocked.

The filter member 55 is arranged in a window opening 57 which is formed in an upper wall 59 of the upper cover 51. The upper cover 51 is provided with a locking protrusion 61 which is adapted for being inserted into a complementary shaped locking opening 63 which is formed in the lower cover 53. Further, the upper cover 51 is provided with screw receiving canals 65 for receiving screws which can be connected to the lower cover 53. The lower cover 53 consequently comprises inner threads for screws (not shown) which may extend through the screw receiving channels 65 to be fixated to the inner threads 67. By this means, the upper cover 51 can be permanently connected to the lower cover 53.

For fixating the outer cage 19 to the heating tube 5, the upper cover 51 comprises upper fixation parts 69 and the lower cover 53 comprises lower fixation parts 71. The upper fixation parts 69 are formed complementary to the lower fixation parts 71 and can, in an assembled state, form fixation means 73 of the outer cage 19. The upper fixation parts 69 and the lower fixation parts 71 are each shaped such that they can receive the heating tube 5 in parts. In other words, the upper fixation parts 69 and the lower fixation parts 71 each comprise the shape of the inner side of a cylinder.

The lower cover 53 comprises a filter member 75. Preferably, the filter member 75 has similar properties as the filter member 55 of the upper cover 51. As the filter member 55 of the upper cover 51, the filter member 75 of the lower cover 53 is preferably arranged in a window opening 77 which is arranged in a lower wall 79 of the lower cover 53.

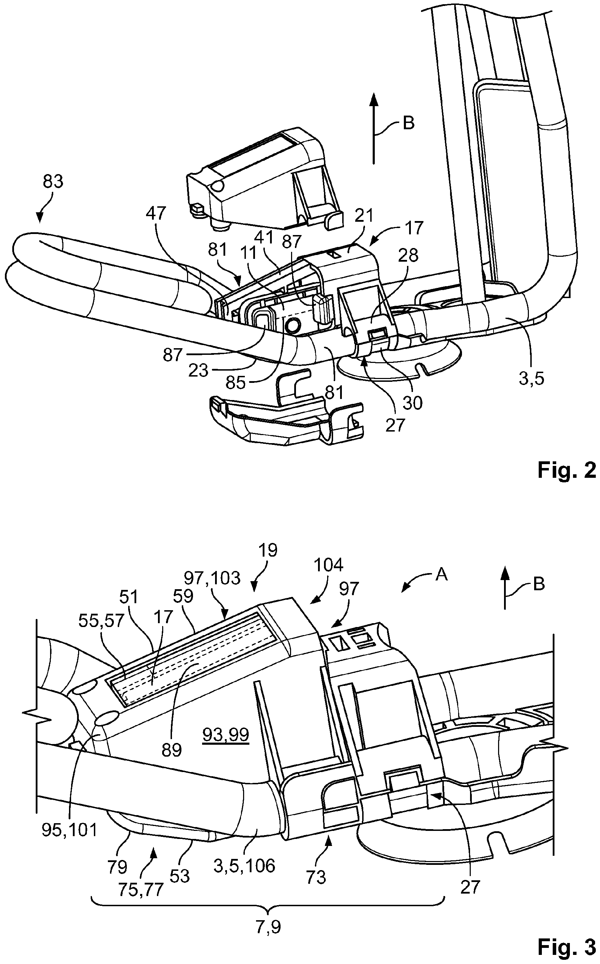

In the following, the urea sensor protection assembly and the urea sensor system are described with respect to Fig. 2.

In Fig. 2, the inner cage 17 is assembled by the connection between the upper part 21 and the lower part 23. The inner cage 17 is fixated to the tubular member 3 is the heating tube 5 by the fixation means 27. The fixation means 27 are formed by connecting the upper fixation part 28 of the upper part 21 with the lower fixation part 30 from the lower part 23. Thereby, the protrusion 39 is received in the recess 37.

Moreover, the latching hook 49 (not shown here) of the arm 47 on the sensor retainer 41 is received in a complementary shaped part of the lower part 23. The fluid sensor 11 is supported by the inner cage 17. Thereby, the upper part 21 with the sensor retainer 41 fixates the fluid sensor 11 on the lower part 23. In a preferred arrangement, the fluid sensor 11 is arranged between two parallel sections 81 of the tubular member 3. Furthermore, the fluid sensor 11 is spaced apart from a loop section 83 of the tubular member 3.

The sensor 11 may comprise at least one optical path 85 which is indicated by a dashed line in Fig. 2. Light transmitted by a light transmitting device of the fluid sensor 11 can travel along the optical path 85 to a light detecting device of the fluid sensor 11. Just by way of example, the fluid sensor 11 is shown with two optical components 87 which can comprise light emitting and/or light detecting devices. The fluid sensor 11 is arranged such that the optical components 87 and the optical path 85 are arranged such that they face the tubular member 3 of the urea sensor system 1. Further, the optical path 85 is basically arranged parallel with said tubular member 3. Consequently, fluid may pass along parallel to the bubble ascension direction B between the optical components 87. Furthermore, gas bubbles may easily leave the area around the optical path 85 without being blocked by an optical component 87.

Fig. 3 shows urea sensor system 7 and the urea sensor protection assembly 9 in the assembled state A. In the assembled state A, the upper cover 51 and the lower cover 53 of the assembly 9 are connected to each other to form the outer cage 19. The inner cage 17 is arranged in an inner volume 89 of the outer cage 19. Consequently, the upper cover 51 and the lower cover 53 jointly form a receptacle 91 for the inner cage 17.

The outer cage 19 is closed at three sides 93, 95 and 97. These sides are preferably formed by solid side walls 99, 101 and 103. Since the outer cage 19 is composed of the upper cover 51 and the lower cover 53, also the side walls 99, 101 and 103 are formed of two parts each. The side wall 99 is arranged opposite to the optical components 87 of the fluid sensor 11 (not shown in Fig. 3). The closed side wall 99 helps to block ambient light such that the fluid sensor 11 is not disturbed by such ambient light. Further, the side wall 99 is preferably formed by a non metallic and/or non reflective material such that also reflections on the side wall 99 are prevented.

Along the bubble ascension direction B, the outer cage 19 is closed by the lower wall 79 and the upper wall 59. As already mentioned, these walls 59 and 79 comprise window openings 57 and 77 with filter members 55 and 75. The filter members 55 and 75 allow fluid to pass through the window openings 57 and 77 and to reach the inner volume 89 of the outer cage 19 such that the fluid can be analyzed by the fluid sensor 11. At a sixth side 104, the outer cage 19 is closed by the inserted inner cage 17.

The inner cage 17 and the outer cage 19 are fixated separately on the tubular member 3, which acts as a common holder 106 for the cages 17 and 19. Thereby, the inner cage 17 is fixated by the fixation means 27 whereas the outer cage 19 is fixated by the fixation means 73. The fixation means 27 and 73 abut each other on the tubular member 3. This abutment may help to align the inner cage 17 and the outer cage 19 relatively to each other. Due to the separate fixation of the inner cage 17 and the outer cage 19, the weight of the outer cage 19 does not have to be carried by the inner cage 17 which carries the fluid sensor 11. Consequently, stress on the inner cage 17 and the fluid sensor 11 is reduced.

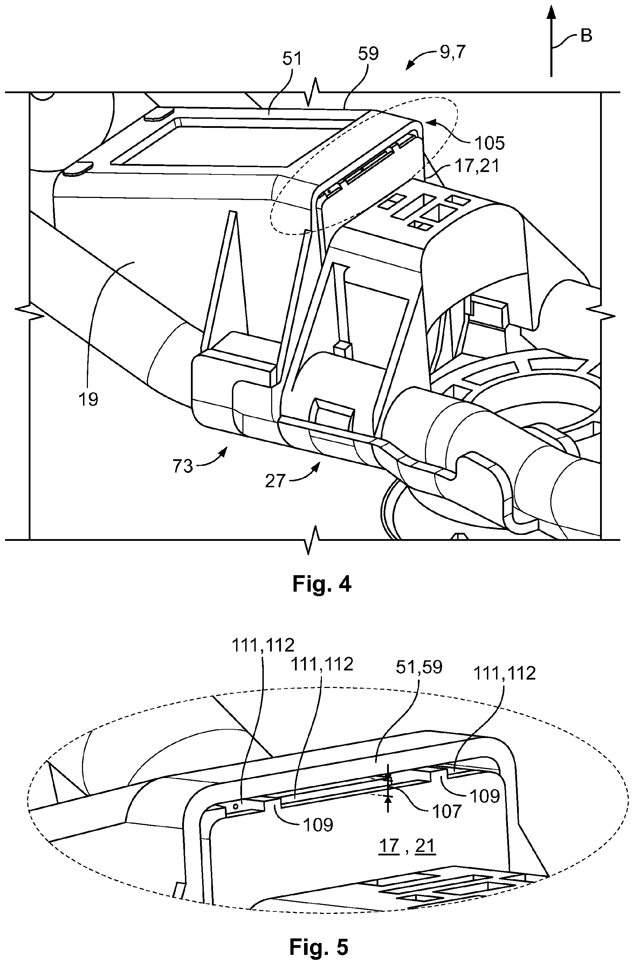

In the following, another advantageous feature of the urea sensor protection assembly 9 according to the invention is described in greater detail with respect to Figs 4 and 5. Thereby, Fig. 5 shows an enlarged view of the encircled region in Fig. 4. For better visibility, the viewing angle on this region is slightly shifted in Fig. 5.

The upper wall 59 of the upper cover 51 is inclined and rises in the bubble ascension B. At the end 105 of the upper wall 59 which faces in the direction of the fixation means 27 of the inner cage 17, the upper wall 59 is spaced apart from the upper part 21 of the inner cage 17.

Thereby, gaps 112 are formed between the upper cover 51 and the upper part 21. In order to keep the distance 107 between the upper wall 59 of the upper cover 51 and the upper part 21 of the inner cage 17, spacers 109 are arranged between the upper part 21 and the upper cover 51. The spacers 109 are formed monolithically with the upper part 21. However, at least one of the spacers 109 may also be formed monolithically with the upper cover 51.

Between the upper part 21 and the upper cover 51, three bubble outlet openings 111 are formed by the gaps 112. The bubble outlet openings 111 allow bubbles to leave the urea sensor protection assembly 9 in order not to disturb the measurements of the fluid sensor 11.

The inclined upper wall 59 may guide bubbles towards the bubble outlet openings 111. However, this is not mandatory. The upper wall 59 may, in the alternative, have a different shape. For example, the upper wall 59 may extend basically perpendicular to the bubble ascension direction B.

The lower wall 79 of the outer cage 19 may be provided with at least one debris outlet opening (not shown) in order to let particles such as debris or ice to leave the outer cage 19 by gravitational force.

### REFERENCE NUMERALS

- 1: urea sensor system
- 3: tubular member
- 5: heating tube
- 7: urea sensor arrangement
- 9: urea sensor protection assembly
- 11: fluid sensor
- 13: fluid tank
- 15: urea tank
- 17: inner cage
- 19: outer cage
- 21: upper part
- 23: lower part
- 25: bottom plate
- 27: fixation means
- 28: upper fixation part
- 29: receptacle
- 30: lower fixation part
- 31: fluid sensor receptacle
- 33: side wall
- 35: bottom wall
- 37: recess
- 39: protrusion
- 41: sensor retainer
- 43: upper wall of the upper part
- 45: through holes
- 47: arm
- 49: latching hook
- 51: upper cover
- 53: lower cover
- 55: filter member
- 57: window opening
- 59: upper wall of the upper cover
- 61: locking protrusion
- 63: locking opening
- 65: screw receiving canal
- 67: inner thread
- 69: upper fixation part
- 71: lower fixation part
- 73: fixation means
- 75: filter member
- 77: window opening
- 79: lower wall
- 81: parallel section
- 83: loop section
- 85: optical path
- 87: optical component
- 89: inner volume of outer cage
- 91: receptacle for inner cage
- 93, 95, 97,104: side
- 99, 101, 103: side walls
- 105: end of upper wall
- 106: common holder
- 107: distance
- 109: spacer
- 111: bubble outlet opening
- 112: gap

- A: assembled state
- B: bubble ascension direction

## Claims

1. Urea sensor protection assembly (9) for protecting at least one fluid sensor (11) of a urea sensor system (1) against bubbles and/or particles wherein the assembly (9) comprises an inner cage (17) for supporting the at least one fluid sensor (11) and an outer cage (19) comprising at least one filter member (55, 75) for blocking air bubbles and/or allowing fluid to pass, wherein the inner cage (17) is, in assembled state (A), at least partially arranged in an inner volume (89) of the outer cage (19), and wherein the inner cage (17) and the outer cage (19) each comprise at least one separate fixation means (27, 73) for separately fixating the corresponding cage (17, 19) to a common holder (106) of the urea sensor system (1).

2. Urea sensor protection assembly (9) according to claim 1, wherein the outer cage (19) is composed of at least one upper cover (51) and at least one lower cover (53), the at least one upper cover (51) and the at least one lower cover (53) jointly forming a receptacle (91) for the inner cage (17).

3. Urea sensor protection assembly (9) according to claim 2, wherein the at least one upper cover (51) and the at least one lower cover (53) each comprise at least one filter member (55, 75).

4. Urea sensor protection assembly (9) according to any of claims 1 to 3, wherein the at least one fixation means (27, 73) is formed clamp-like for being fixated to a tubular part (3, 5) of the sensor system (1).

5. Urea sensor protection assembly (9) according to any of claims 1 to 4, wherein the urea protection assembly (9) is further provided with at least one bubble outlet opening (111).

6. Urea sensor protection assembly (9) according to claim 5, wherein the at least one bubble outlet opening (111) is arranged above the inner cage (17) in a bubble ascension direction (B).

7. Urea sensor protection assembly (9) according to claim 5 or 6, wherein the at least one bubble outlet opening (111) is formed by a gap (112) between the inner cage (17) and the outer cage (19).

8. Urea sensor protection assembly (9) according to claim 7, wherein the inner cage (17) and the outer cage (19) are spaced apart by at least one spacer (109) to form the at least one bubble outlet opening (111).

9. Urea sensor protection assembly (9) according to any of claims 5 to 8, wherein the upper cover (51) has (a) an upper wall (59) which extends perpendicular to the bubble ascension direction (B) towards the at least one bubble outlet opening (111) or (b) an inclined upper wall (59) which rises in the bubble ascension direction (B) towards the at least one bubble outlet opening (111).

10. Urea sensor protection assembly (9) according to any of claims 1 to 9, wherein the outer cage (19) is closed at at least three sides (93, 95, 97), wherein the inner cage (17) is inserted in another side (104).

11. Urea sensor protection assembly (9) according to claim 10, wherein the at least three sides (93, 95, 97) are formed by solid side walls (99, 101, 103).

12. Urea sensor arrangement (7) comprising at least one urea sensor protection assembly (9) according to any of claims 1 to 11 and at least one fluid sensor (11), wherein the at least one fluid sensor (11) is arranged inside the inner cage (17) of the urea sensor protection assembly (9).

13. Urea sensor protection system (1) comprising at least one tubular member (3, 5) for being arranged in a fluid tank (13), and at least one urea sensor protection assembly (9) according to any of claims 1 to 11, wherein the inner cage (17) and the outer cage (19) of the urea sensor protection assembly (9) are fixated to the at least one tubular member (3).

14. Urea sensor protection system (1) according to claim 13, wherein the at least one fixation means (27) of the inner cage (17) abuts the at least one fixation means (73) of the outer cage (19) on the tubular member (3).

15. Urea sensor protection system (1) according to claim 13 or 14, wherein at least the inner cage (17) is arranged between two parallel sections (81) of the at least one tubular member (3).

## Patentansprüche

1. Harnstoffsensor-Schutzanordnung (9) zum Schutz wenigstens eines Fluidsensors (11) eines Harnstoffsensorsystems (1) gegen Blasen und/oder Partikel, wobei die Anordnung (9) einen Innenkäfig (17) zum Halten des wenigstens einen Fluidsensors (11) und einen äußeren Käfig (19) mit wenigstens einem Filterelement (55, 75) zum Blockieren von Luftblasen und/oder zum Durchlassen von Fluid umfasst, wobei der innere Käfig (17) im zusammengebauten Zustand (A) wenigstens teilweise in einem Innenvolumen (89) des Außenkäfigs (19) angeordnet ist und der Innenkäfig (17) und der Außenkäfig (19) jeweils wenigstens eine separate Befestigungseinrichtung (27, 73) zur separaten Befestigung des entsprechenden Käfigs (17, 19) an einer gemeinsamen Halterung (106) des Harnstoffsensorsystems (1) aufweisen.

2. Harnstoffsensor-Schutzanordnung (9) nach Anspruch 1, bei der der äußere Käfig (19) aus wenigstens einer oberen Abdeckung (51) und wenigstens einer unteren Abdeckung (53) besteht, wobei die wenigstens eine obere Abdeckung (51) und die wenigstens eine untere Abdeckung (53) gemeinsam eine Aufnahme (91) für den inneren Käfig (17) bilden.

3. Harnstoffsensor-Schutzanordnung (9) nach Anspruch 2, bei der die wenigstens eine obere Abdeckung (51) und die wenigstens eine untere Abdeckung (53) jeweils wenigstens ein Filterelement (55, 75) umfassen.

4. Harnstoffsensor-Schutzanordnung (9) nach einem der Ansprüche 1 bis 3, bei der die wenigstens eine Befestigungseinrichtung (27, 73) klemmenartig ausgebildet ist, um an einem rohrförmigen Teil (3, 5) des Sensorsystems (1) befestigt zu werden.

5. Harnstoffsensor-Schutzanordnung (9) nach einem der Ansprüche 1 bis 4, bei der die Harnstoffschutzanordnung (9) weiterhin mit wenigstens einer Blasenauslassöffnung (111) versehen ist.

6. Harnstoffsensor-Schutzanordnung (9) nach Anspruch 5, bei der die wenigstens eine Blasenauslassöffnung (111) in Blasenaufstiegsrichtung (B) oberhalb des Innenkäfigs (17) angeordnet ist.

7. Harnstoffsensor-Schutzanordnung (9) nach Anspruch 5 oder 6, bei der die wenigstens eine Blasenauslassöffnung (111) durch einen Spalt (112) zwischen dem Innenkäfig (17) und dem Außenkäfig (19) ausgebildet ist.

8. Harnstoffsensor-Schutzanordnung (9) nach Anspruch 7, bei der der innere Käfig (17) und der äußere Käfig (19) durch wenigstens einen Abstandshalter (109) voneinander beabstandet sind, um die wenigstens eine Blasenauslassöffnung (111) auszubilden.

9. Harnstoffsensor-Schutzanordnung (9) nach einem der Ansprüche 5 bis 8, bei der die obere Abdeckung (51) (a) eine obere Wand (59), die sich senkrecht zur Blasenaufstiegsrichtung (B) zu der wenigstens einen Blasenauslassöffnung (111) erstreckt, oder (b) eine geneigte obere Wand (59) aufweist, die in Blasenaufstiegsrichtung (B) zu der wenigstens einen Blasenauslassöffnung (111) ansteigt.

10. Harnstoffsensor-Schutzanordnung (9) nach einem der Ansprüche 1 bis 9, bei der der äußere Käfig (19) an wenigstens drei Seiten (93, 95, 97) geschlossen ist, wobei der innere Käfig (17) an einer weiteren Seite (104) eingefügt ist.

11. Harnstoffsensor-Schutzanordnung (9) nach Anspruch 10, bei der die wenigstens drei Seiten (93, 95, 97) durch massive Seitenwände (99, 101, 103) ausgebildet sind.

12. Harnstoffsensoranordnung (7), umfassend wenigstens eine Harnstoffsensor-Schutzanordnung (9) nach einem der Ansprüche 1 bis 11 und wenigstens einen Fluidsensor (11), wobei der wenigstens eine Fluidsensor (11) innerhalb des inneren Käfigs (17) der Harnstoffsensor-Schutzanordnung (9) angeordnet ist.

13. Harnstoffsensor-Schutzsystem (1), umfassend wenigstens ein rohrförmiges Element (3, 5) zur Anordnung in einem Fluidtank (13) und wenigstens eine Harnstoffsensor-Schutzanordnung (9) nach einem der Ansprüche 1 bis 11, wobei der innere Käfig (17) und der äußere Käfig (19) der Harnstoffsensor-Schutzanordnung (9) an dem wenigstens einen rohrförmigen Element (3) befestigt sind.

14. Harnstoffsensor-Schutzsystem (1) nach Anspruch 13, bei der die wenigstens eine Befestigungseinrichtung (27) des Innenkäfigs (17) an der wenigstens einen Befestigungseinrichtung (73) des Außenkäfigs (19) an dem rohrförmigen Element (3) anliegt.

15. Harnstoffsensor-Schutzsystem (1) nach Anspruch 13 oder 14, wobei wenigstens der Innenkäfig (17) zwischen zwei parallelen Abschnitten (81) des wenigstens einen rohrförmigen Elementes (3) angeordnet ist.

## Revendications

1. Ensemble de protection de capteur d'urée (9) pour protéger au moins un capteur de fluide (11) d'un système de capteur d'urée (1) contre les bulles et/ou les particules, dans lequel l'ensemble (9) comprend une cage intérieure (17) pour supporter ledit au moins un capteur de fluide (11) et une cage extérieure (19) comprenant au moins un élément filtre (55, 75) pour bloquer les bulles d'air et/ou laisser passer le fluide, dans laquelle la cage intérieure (17) est, à l'état assemblé (A), au moins partiellement disposée dans un volume intérieur (89) de la cage extérieure (19), et dans laquelle la cage intérieure (17) et la cage extérieure (19) comprennent chacune au moins un moyen de fixation séparé (27, 73) pour fixer séparément la cage correspondante (17, 19) à un support commun (106) du système (1) de capteur d'urée.

2. Ensemble de protection de capteur d'urée (9) selon la revendication 1, dans lequel la cage extérieure (19) est composée d'au moins un couvercle supérieur (51) et d'au moins un couvercle inférieur (53), ledit au moins un couvercle supérieur (51) et ledit au moins un couvercle inférieur (53) formant ensemble un logement (91) de la cage intérieure (17).

3. Ensemble de protection de capteur d'urée (9) selon la revendication 2, dans lequel ledit au moins un couvercle supérieur (51) et ledit au moins un couvercle inférieur (53) comprennent chacun au moins un élément filtrant (55, 75).

4. Ensemble de protection de capteur d'urée (9) selon l'une quelconque des revendications 1 à 3, dans lequel ledit au moins un moyen de fixation (27, 73) est en forme de pince pour être fixé à une partie tubulaire (3, 5) du système de capteur (1).

5. Ensemble de protection de capteur d'urée (9) selon l'une quelconque des revendications 1 à 4, dans lequel l'ensemble de protection de capteur d'urée (9) est en outre pourvu d'au moins une ouverture de sortie de bulles (111).

6. Dispositif de protection de capteur d'urée (9) selon la revendication 5, dans lequel ladite au moins une ouverture de sortie de bulles (111) est disposée au-dessus de la cage intérieure (17) dans une direction de montée de bulles (B).

7. Ensemble de protection de capteur d'urée (9) selon les revendications 5 ou 6, dans lequel ladite au moins une ouverture de sortie de bulles (111) est formée par un espace (112) entre la cage intérieure (17) et la cage extérieure (19).

8. Ensemble de protection de capteur d'urée (9) selon la revendication 7, dans lequel la cage intérieure (17) et la cage extérieure (19) sont espacées d'au moins une entretoise (109) pour former ladite au moins une ouverture de sortie de bulle (111).

9. Ensemble de protection de capteur d'urée (9) selon l'une quelconque des revendications 5 à 8, dans lequel le couvercle supérieur (51) présente (a) une paroi supérieure (59) qui s'étend perpendiculairement à la direction d'ascension de bulles (B) vers ladite au moins une ouverture de sortie de bulles (111) ou (b) une paroi supérieure inclinée (59) qui monte dans la direction d'ascension de bulles (B) vers ladite au moins une ouverture de sortie de bulles (111).

10. Ensemble de protection de capteur d'urée (9) selon l'une quelconque des revendications 1 à 9, dans lequel la cage extérieure (19) est fermée sur au moins trois côtés (93, 95, 97), la cage intérieure (17) étant introduite dans un autre côté (104).

11. Ensemble de protection de capteur d'urée (9) selon la revendication 10, dans lequel lesdits au moins trois côtés (93, 95, 97) sont formés par des parois latérales solides (99, 101, 103).

12. Agencement de capteur d'urée (7) comprenant au moins un ensemble de protection de capteur d'urée (9) selon l'une quelconque des revendications 1 à 11 et au moins un capteur de fluide (11), dans lequel ledit au moins un capteur de fluide (11) est disposé à l'intérieur de la cage intérieure (17) de l'ensemble de protection de capteur d'urée (9).

13. Système de protection de capteur d'urée (1) comprenant au moins un élément tubulaire (3, 5) destiné à être disposé dans un réservoir de fluide (13), et au moins un ensemble de protection de capteur d'urée (9) selon l'une quelconque des revendications 1 à 11, dans lequel la cage intérieure (17) et la cage extérieure (19) de l'ensemble de protection de capteur d'urée (9) sont fixées au moins un élément tubulaire (3).

14. Système de protection de capteur d'urée (1) selon la revendication 13, dans lequel ledit au moins un moyen de fixation (27) de la cage intérieure (17) est adjacent audit au moins un moyen de fixation (73) de la cage extérieure (19) sur l'élément tubulaire (3).

15. Système de protection de capteur d'urée (1) selon les revendications 13 ou 14, dans lequel au moins la cage intérieure (17) est disposée entre deux sections parallèles (81) dudit au moins un élément tubulaire (3).
